(19) Europäisches Patentamt
European Patent Office
Office européen des brevets

(11) EP 2 671 866 A1

(12) **EUROPÄISCHE PATENTANMELDUNG**

(43) Veröffentlichungstag:
**11.12.2013 Patentblatt 2013/50**

(21) Anmeldenummer: **12170697.2**

(22) Anmeldetag: **04.06.2012**

(51) Int Cl.:
***C07C 209/68*** (2006.01) ***C07B 37/04*** (2006.01)
***C07B 49/00*** (2006.01) ***C07D 317/50*** (2006.01)
***C07C 1/32*** (2006.01) ***C07C 41/30*** (2006.01)
***C07C 17/263*** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Saltigo GmbH**
**51369 Leverkusen (DE)**

(72) Erfinder: **Die Erfindernennung liegt noch nicht vor**

(74) Vertreter: **Pettrich, Klaus-Günter**
**LANXESS Deutschland GmbH**
**LIP-IPR**
**LANXESS Tower**
**Kennedyplatz 1**
**50569 Köln (DE)**

(54) **Verfahren zur Herstellung von Styrolderivaten**

(57) Es wird ein Verfahren bereitgestellt, welches die Synthese einer großen Anzahl an Styrolderivaten unter Bildung von C-C Bindungen ermöglicht, wobei ökonomisch vorteilhafte Substrate, leicht zugängliche Kohlenstoffnucleophile und sowohl kostengünstige als auch ökologisch unproblematische Katalysatorsysteme eingesetzt werden können, welche eine Reaktion unter milden Bedingungen und eine große Kompatibilität mit funktionellen Gruppen an den beteiligten Reaktionspartnern erlauben.

EP 2 671 866 A1

## Beschreibung

Gegenstand der Erfindung ist ein Verfahren zur Herstellung von Styrolderivaten durch übergangsmetallkatalysierte Kreuzkupplung von Chlorstyrolen mit Organomagnesiumverbindungen in Gegenwart von Eisen-Verbindungen.

Übergangsmetallkatalysierte Kreuzkupplungen sind wichtige Synthesewerkzeuge der modernen organischen Chemie, wobei jedoch bei der Mehrzahl der bekannten Kreuzkupplungsreaktionen Palladium- oder Nickel-Komplexe als Übergangsmetall-Katalysatoren verwendet werden. Die Kreuzkupplung mit ökonomisch und ökologisch besonders vorteilhaften Chlorstyrolen unter Substitution des Chloratoms ist nur in wenigen Einzelfällen beschrieben, zum Beispiel als Arylierung aktivierter Benzyl-Derivate durch Niwa et al., Org. Lett. 2008, 10, 4639, als *Sonogashira*-Kupplung mit terminalen Alkinen durch Torborg et al., Chem. Eur. J. 2009, 15, 1329, und als *Suzuki*-Kupplung mit Arylboronsäuren, durch Lietzau et al., WO 2006/125511, wobei sämtliche Reaktionen palladiumkatalysiert abliefen. Zudem zeigten sich die bekannten Reaktionen dieser Klasse entweder als sehr substratspezifisch in Bezug auf den Kupplungspartner (z. B. beschränkt auf CH-azide Benzylderivate bzw. terminale Alkine) oder benötigten aufwendige und kostspielige Borsäurederivate als Kupplungspartner. Um eine akzeptable Reaktivität des Katalysatorssystems zu erzielen, war außerdem die Verwendung von organischen Liganden in Form von Phosphinen oder N-heterocyclischen Carbenen erforderlich, welche jedoch relativ teuer und in der Regel nicht rückgewinnbar sind. Ferner war eine Kreuzkupplung mit einfachen Alkyl-Derivaten unter Verwendung dieser Katalysatoren nicht möglich.

Daher wurde versucht, Verfahren zur Kreuzkupplung zu entwickeln, welche sowohl die Verwendung der ökonomisch vorteilhaften Chlorstyrole erlauben als auch den Einsatz von leicht zugänglichen Kohlenstoffnucleophilen, wie z.B. Grignard-Verbindungen, wobei nicht zuletzt kostengünstige, leicht verfügbare und ungiftige Katalysatoren und Liganden zur Anwendung kommen sollten.

Eisenverbindungen sind zu wesentlich günstigeren Preisen verfügbar als Palladium, weit weniger toxisch als Nickelverbindungen und bieten vom ökologischen Standpunkt deutliche Vorteile gegenüber auf Kupfer basierenden Katalysatorsystemen. Insbesondere die Aufreinigung von Abwässern wird deutlich erleichtert.

Bereits zu Beginn der 1970er Jahre wurde gefunden, dass Eisensalze die Kreuzkupplung von Vinylhalogeniden mit Alkylgrignard-Verbindungen katalysieren können (Kochi et al., J. Am. Chem. Soc. 1971, 1487), jedoch fand diese Reaktion aufgrund der geringen Anwendungsbreite in den folgenden 30 Jahren nur sehr begrenzte Anwendung, bis es *Knochel, Fürstner, Cahiez* und *Nakamura* gelang, eisenkatalysierte Kreuzkupplungen mit Hilfe von stickstoffhaltigen Additiven wie z.B. N-Methylpyrrolidon oder N,N,N',N'-Tetramethylethylendiamin (TMEDA) auf eine größere Substratbreite anzuwenden (z.B. Fürstner et al., Angew. Chem. Int. Ed. 2002, 41, 609; Nakamura et al., J. Am. Chem. Soc. 2004, 3686; Knochel et al., Synlett 2001, 1901; Cahiez et al., Angew. Chem. Int. Ed. 2007, 4364). Diese Reaktionen zeichnen sich durch besonders milde Reaktionsbedingungen (-20 °C bis +35 °C), hohe Funktionsgruppenkompatibilität (z.B. Methylester, Amine) und kurze Reaktionszeiten (meist weniger als zwei Stunden) aus. Besonders interessant für die industrielle Anwendung sind diese Reaktionen auch deshalb, weil sie im Regelfall keine teuren und empfindlichen Phosphin- oder Carben-Liganden benötigen, wie es bei Nickel- und Palladium-basierten Katalysatorsystemen häufig der Fall ist, insbesondere wenn preiswerte Arylchloride anstelle der -bromide oder -iodide als Kupplungspartner dienen.

Diese eisenkatalysierten Kreuzkupplungen galten jedoch als beschränkt auf elektronenarme Chlorstyrole, bei denen der Arylring elektronenziehende Carboxyl-, Cyano-, Trifluormethyl-, oder Sulfoxylsubstituenten aufwies, bzw. auf elektronenarme chlorierte heteroaromatische Verbindungen. Fürstner et. al. lehrten folglich, bei elektronenschiebenden Substituenten am Aromaten, wie Methyl, Methoxy, Aryl, oder Aryloxy, auf die reaktiveren, wenngleich synthetisch schwieriger zugänglichen Aryltriflate zurückzugreifen. Die Kreuzkupplung von aufgrund der Alkenylgruppe relativ elektronenreichen Chlorstyrole mit Organomagnesiumverbindungen wurde weder durchgeführt noch versucht.

Die Aufgabe der vorliegenden Erfindung bestand darin, ein Verfahren zur Herstellung von Styrolderivaten durch Kreuzkupplung bereitzustellen, bei dem preisgünstige und umweltfreundliche Eisenkatalysatoren verwendet werden können, um leicht zugängliche und mit großer Substratbreite herstellbare Organomagnesium-Verbindungen mit Chlorstyrolen zu verknüpfen.

Die vorliegende Aufgabe konnte gelöst werden, indem überraschenderweise gefunden wurde, dass trotz der elektronenschiebenden Wirkung der Alkenylgruppe, Chlorstyrole in der Anwesenheit von Eisenverbindungen mit Magnesiumorganylen kreuzgekuppelt werden können, wobei typischerweise die bekannten milden Bedingungen der Eisenkatalyse angewendet werden können. Dadurch wird ein effizienter, kostengünstiger und umweltfreundlicher neuer Zugang zu einer Substanzklasse geschaffen, die bisher nur wesentlich aufwendiger zugänglich war.

Gegenstand der Erfindung ist daher ein Verfahren zur Herstellung organischer Verbindungen der allgemeinen Formel (I)

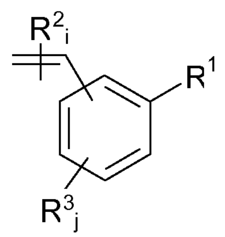

(I),

worin

- i für 0, 1, 2 oder 3, bevorzugt für 0 oder 1 steht,
- j für 0, 1, 2, 3 oder 4, bevorzugt für 0 oder 1 steht,
- $R^2$ unabhängig voneinander ausgewählt ist aus unsubstituiertem, oder mit Fluor, $NR^4_2$, $C_1$-$C_6$-Alkyl oder $C_1$-$C_6$-Alkoxy-substituiertem $C_1$-$C_6$-Alkyl, $C_6$-$C_{10}$-Aryl oder $C_7$-$C_{11}$-Aralkyl, wobei jedes $R^4$ unabhängig voneinander, für $C_1$-$C_6$-Alkyl, $C_7$-$C_{10}$-Aryl oder $C_7$-$C_{11}$-Aralkyl stehen kann,
- $R^3$ unabhängig voneinander ausgewählt ist aus Fluor, Chlor, $NR^4_2$, unsubstituiertem, oder mit $NR^4_2$ oder $C_1$-$C_6$-Alkoxy-substituiertem $C_1$-$C_6$-Alkyl, sowie unsubstituiertem, oder mit Fluor, $NR^4_2$ oder $C_1$-$C_6$-Alkoxy-substituiertem $C_1$-$C_6$-Alkoxy oder $C_7$-$C_{11}$-Aralkyl, wobei $R^4$ jeweils unabhängig voneinander die oben angegebenen Bedeutungen hat, wobei zwei oder mehr der Substituenten $R^2$ und/oder $R^3$ zusammen ein aromatisches, heteroaromatisches, aliphatisches oder heteroaliphatisches Ringsystem bilden, unter der Bedingung, dass die den Rest $R^2$ tragende Vinylfunktion nicht Bestandteil eines solchen Ringsystems ist, sofern dieses aromatisch oder heteroaromatisch ist, und
- $R^1$ ein unsubstituierter, oder mit Fluor, $C_1$-$C_{10}$-Alkyl, $C_1$-$C_6$-Alkoxy, oder $NR^4_2$, substituierter $C_1$-$C_{15}$-Alkyl, $C_3$-$C_7$-Cycloalkyl, $C_2$-$C_{15}$-Alkenyl, $C_3$-$C_7$-Cycloalkenyl, $C_2$-$C_{15}$-Alkinyl, $C_1$-$C_6$-Alkoxy-, $C_7$-$C_{15}$-Aralkyl-, oder $C_6$-$C_{10}$-Aryl-Rest, oder ein entsprechend substituierter oder unsubstituierter aliphatischer oder aromatischer $C_3$-$C_{12}$-Heterocyclus ist, wobei $R^4$ jeweils unabhängig voneinander die oben angegebenen Bedeutungen hat,

durch Umsetzung von Chlorstyrolen der allgemeinen Formel (II)

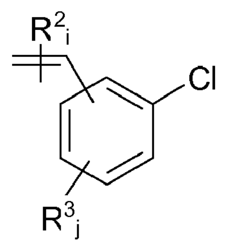

(II),

worin
$R^2$, $R^3$, i und j die für Formel (I) beschriebenen Bedeutungen haben, mit Organomagnesiumverbindungen der formalen Zusammensetzung (III)

$$[M+]_n\ [R_mMgX_kY_1] \qquad (III),$$

worin

- $R^1$ die für Formel (I) beschriebene Bedeutung hat,
- M für Lithium, Natrium oder Kalium steht,
- X für Fluor, Chlor, Brom oder lod, bevorzugt Chlor oder Br Chlor, Brom, ganz besonders bevorzugt für Brom steht,
- Y für Fluor, Chlor, Brom oder lod, bevorzugt Chlor oder Br Chlor, Brom, ganz besonders bevorzugt für Brom steht,
- n für 0, 1, 2, 3 oder 4, bevorzugt 0 oder 1, ganz besonders bevorzugt für 0 steht,
- m für 1, 2, 3, 4, 5 oder 6, bevorzugt 1 oder 2, ganz besonders bevorzugt für 1 steht,
- k für 0, 1, 2, 3 oder 4, bevorzugt 0, 1 oder 2, ganz besonders bevorzugt für 0 oder 1 steht,
- l ür 0, 1, 2, 3 oder 4 bevorzugt 0, 1 oder 2, ganz besonders bevorzugt für 0 oder 1 steht,

und gleichzeitig die Beziehung

$$n + 2 = m + k + l$$

gilt,
dadurch gekennzeichnet, dass die Umsetzung in Gegenwart von Eisenverbindungen, bevorzugt Eisen(II)- oder Eisen (III)-Verbindungen durchgeführt wird.

In einer bevorzugten Ausführungsform steht i für 0 oder 1. In einer weiteren bevorzugten Ausführungsform ist j 0,1 oder 2, wobei in einer besonders bevorzugten Ausführungsform i für 0 oder 1 und j für 0,1 oder 2 steht.

In einer bevorzugten Ausführungsform ist $R^2$ unabhängig voneinander ausgewählt aus $C_1$-$C_6$-Alkyl, $C_6$-$C_{10}$-Aryl, und $C_7$-$C_{11}$-Aralkyl, wobei Methyl, Ethyl oder Propyl, Phenyl und Naphthyl besonders bevorzugte Ausführungsformen darstellen.

In einer bevorzugten Ausführungsform wird $R^3$ unabhängig voneinander ausgewählt aus Chlor, $NR^4{}_2$, wobei jedes $R^4$ unabhängig voneinander, für $C_1$-$C_6$-Alkyl, $C_7$-$C_{10}$-Aryl oder $C_7$-$C_{11}$-Aralkyl, bevorzugt $C_1$-$C_6$-Alkyl stehen kann, $C_1$-$C_6$-Alkyl, besonders bevorzugt Methyl, Ethyl oder Propyl, $C_1$-$C_6$-Alkoxy, $C_6$-$C_{10}$-Aryl, besonders bevorzugt Phenyl oder Naphthyl und $C_7$-$C_{11}$-Aralkyl.

Bei der Ausführungsform, bei welcher zwei oder mehr der Substituenten $R^2$ und/oder $R^3$ zusammen ein heteroaromatisches oder heteroaliphatisches Ringsystem bilden sind Stickstoff, Sauerstoff oder Schwefel und ganz besonders Sauerstoff als Heteroatome des heteroaliphatischen oder (hetero)araliphatischen Ringsystems bevorzugt, wobei die Heteroatome beim heteroaraliphatischen Ringsystem vorzugsweise im aliphatischen Teil des Ringsystems vorliegen.

Im Sinne der vorliegenden Erfindung umfasst der aliphatische oder aromatische heterocyclische $C_3$-$C_{12}$-Rest beim Rest $R^1$ mono- und polycyclische Verbindungen, bei denen zumindest ein Ring als Heterocyclus vorliegt und ggf. vorhandene weitere Ringe aus Kohlenwasserstoffen und/oder Heteroatomen aufgebaut sind, wobei die Heteroatome des aliphatischen oder aromatischen heterocyclischen $C_3$-$C_{12}$-Rests vorzugsweise ausgewählt sind aus Sauerstoff, Stickstoff und/oder Schwefel.

In einer bevorzugten Ausführungsform ist $R^1$ ein unsubstituierter, oder mit Fluor, $C_1$-$C_4$-Alkyl, $C_1$-$C_4$-Alkoxy oder $NR^4{}_2$, substituierter $C_1$-$C_{11}$-Alkyl, $C_3$-$C_7$-Cycloalkyl, $C_2$-$C_{11}$-Alkenyl, $C_3$-$C_7$-Cycloalkenyl, $C_2$-$C_{11}$-Alkinyl, $C_7$-$C_{12}$-Aralkyl-, oder $C_6$-$C_{10}$-Aryl-Rest, oder ein entsprechend substituierter oder unsubstituierter aliphatischer oder aromatischer $C_3$-$C_8$-Heterocyclus, wobei $R^4$ jeweils unabhängig voneinander $C_1$-$C_6$-Alkyl ist. Der aliphatische oder aromatische heterocyclische $C_3$-$C_8$-Rest umfasst dabei mono- und polycyclische Verbindungen, bei denen zumindest ein Ring als Heterocyclus vorliegt und ggf. vorhandene weitere Ringe aus Kohlenwasserstoffen und/oder Heteroatomen aufgebaut sind, wobei die Heteroatome des aliphatischen oder aromatischen heterocyclischen $C_3$-$C_8$-Rests aus Sauerstoff und/oder Stickstoff, bevorzugt aus Sauerstoff bestehen.

Chlorstyrole der allgemeinen Formel (II) sind kommerziell erhältlich oder mittels bekannter Methoden wie z. B. Wittig-Olefinierung oder Heck-Reaktion herstellbar. Erfindungsgemäß werden sie bevorzugt einzeln eingesetzt, wobei jedoch auch die Verwendung einer Mischung von zwei oder mehr Chlorstyrolen möglich ist. Die Alkenylgruppe des Chlorstyrols steht dabei in ortho-, meta- oder para-, vorzugsweise ortho- oder para- und besonders bevorzugt in ortho-Position zum Chlorsubstituenten.

Beispiele für Chlorstyrole der Formel (II) sind 2-Chlorstyrol, 3-Chlorstyrol, 4-Chlorstyrol, 4-Chlorstilben, 1-Chlor-4-isopropenylbenzol, 1-Chlor-3-isopropenylbenzol, 4-N,N-Dimethylamino-2-chlorstyrol, 3-Methoxy-2-chlorstyrol, 2-Chlor-4,5-methylendioxystyrol, 2-Chlor-4,5-dimethoxystyrol, 4-(4-Chlorphenyl-3-methylprop-1-en)-1-ylstyrol, 2,4-Dichlorstyrol und 3-N,N-Dimethylamino-2-chlorstyrol. 1.

Die Herstellung der Organomagnesiumverbindung (III) ist dem Fachmann geläufig, z.B. durch Grignard-Reaktion einer organischen Halogenverbindung R-X (wobei X für Fluor, Chlor, Brom, Iod steht) mit elementarem Magnesium, unter geeigneten Bedingungen auch durch Halogen-Metall-Austausch oder Deprotonierung, optional unter Zusatz von Hilfsstoffen wie z.B. Lithiumchlorid, oder durch Transmetallierung anderer Organometallverbindungen, z.B. Organolithiumverbindungen, mit geeigneten Magnesiumverbindungen, z.B. Magnesiumsalzen oder Grignard-Verbindungen. Für das erfindungsgemäße Verfahren werden bevorzugt Organomagnesiumchloride $R^1MgCl$ oder Organomagnesiumbromide $R^1MgBr$, ganz besonders bevorzugt Organomagnesiumbromide $R^1MgBr$ eingesetzt, wobei $R^1$ die für Formel (I) beschriebene Bedeutung hat

Beispiele für geeignete Organomagnesiumverbindungen sind Organomagnesiumbromide wie z. B. Nonylmagnesiumbromid, Undecylmagnesiumbromid, 2-Ethylhexyl-1-magnesiumbromid, Cyclopropylmagnesiumbromid, 2-Cyclohexylethyl-1-magnesiumbromid, 2-Propylendioxyethyl-1-magnesiumbromid, 9-Decen-1-ylmagnesiumbromid, 6-Tetrahydropyran-2-oxyhexyl-1-magnesium-bromid, 4-(N,N-Dimethylamino)-phenylmagnesiumbromid, 4'-Fluorbiphenyl-4-magnesium-bromid, 4-Fluorphenylmagnesiumbromid, 4-Methoxyphenylmagnesiumbromid, 4-Fluor-3-methylphenylmagnesiumbromid und Thiophen-2-ylmagnesiumbromid; sowie Bis(4-methoxyphenyl)-magnesium, Bis(4-Methoxyphenyl)-magnesium-Lithiumchlorid-Komplex, 4-Methoxyphenylmagnesiumchlorid-Lithiumchlorid-Komplex und Lithiumtrihexylmagnesat.

Das bevorzugte molare Verhältnis von eingesetztem Chlorstyrol der Formel (II) zu Organomagnesiumverbindung (III) beträgt von 10:1 bis 1:10, besonders bevorzugt von 3:1 bis 1: 3 und ganz besonders bevorzugt von 3:2 bis 2:3.

Das erfindungsgemäße Verfahren wird üblicherweise in einem trockenen aprotischen organischen Lösungsmittel oder einer Mischung von einem oder mehreren dieser Lösungsmittel durchgeführt. In einer bevorzugten Ausführungsform enthält oder besteht das Lösungsmittel aus einem oder mehreren Ethern, bevorzugt ausgewählt aus Tetrahydrofuran (THF), 2-Methyltetrahydrofuran (2-Methyl-THF), 1,4-Dioxan, Methyl-tert-butylether (MTBE), Diethylether, 1,2-Dimethoxyethan (DME, Glyme), Diisopropylether (DIPE), Dipropylether, Dibutylether, Cyclopentylmethylether, Diethylenglycoldimethylether (Diglyme), Triethylenglycoldimethylether (Triglyme), Tetraethylenglycoldimethylether (Tetraglyme) und Diethylenglycoldibutylether, wobei THF besonders bevorzugt ist.

In einer bevorzugten Ausführungsform wird die Reaktion in einem Lösungsmittel durchgeführt, welches ein oder mehrere stickstoffhaltige Additive, ausgewählt aus Trialkylaminen, vorzugsweise Triethylamin, Ethyldiisopropylamin oder N,N,N',N'-Tetramethylethylendiamin (TMEDA), N-haltigen aliphatischen Heterocyclen, vorzugsweise 1,4-Diazabicyclo[2.2.2]octan (DABCO) oder Spartein, Alkylamide, cyclischen Alkylamiden (Lactamen), vorzugsweise N-Methyl-2-pyrrolidon (NMP), Cycloalkylaminen, vorzugsweise 1,2-Diaminocyclohexan (DACH), Cycloalkyldiaminen, Alkyliminen, Cycloalkyliminen, Anilin-Derivaten, vorzugsweise N,N-Dimethylanilin, Harnstoffen, Urethanen oder stickstoffhaltigen Heteroaromaten, vorzugsweise Pyridin oder Phenanthrolin, enthält. Besonders bevorzugt wird N-Methyl-2-pyrrolidin (NMP) zugesetzt. Das Additiv kann zudem die Rolle eines Cosolvents übernehmen.

Im erfindungsgemäßen Verfahren beträgt der Anteil des stickstoffhaltigen Additivs bezogen auf die eingesetzte Lösungsmittelmenge 0.1 bis 50 Vol.-%, besonders bevorzugt 1 bis 20 Vol.-%, ganz besonders bevorzugt 5-15 Vol.-%.

Die Eisenverbindung kann bei der vorliegenden Erfindung in beliebiger Oxidationsstufe eingesetzt werden, wobei aus praktischen Gründen Verbindungen mit Eisen in der Oxidationsstufe +2 oder +3, z.B. Eisen(II)chlorid, Eisen (III)chlorid, Eisen(II)acetylacetonat, Eisen(III)acetylacetonat, Eisen(II)acetat, Eisen(III)acetat, Eisen(II)bromid, Eisen(III) bromid, Eisen(II)fluorid, Eisen(III)fluorid, Eisen(II)iodid, Eisen(III)iodid, Eisen(II)sulfat, Eisen(II)trifluoracetat, Eisen(II) trifluormethansulfonat, Eisen(III)trifluormethansulfonat, Eisen(III)chlorid-TMEDA-Komplex oder eine Mischung dieser Verbindungen bevorzugt sind.

Die Eisenverbindung kann in substöchiometrischer, stöchiometrischer oder überstöchiometrischer Menge eingesetzt werden, wobei bevorzugt eine substöchiometrische Menge, besonders bevorzugt eine Menge von 0,01 bis 20 mol% und ganz besonders bevorzugt eine Menge von 0,01 bis 10 mol% bezogen auf die Verbindung der allgemeinen Formel (II) Verwendung findet.

Das erfindungsgemäße Verfahren wird üblicherweise bei einer Reaktionstemperatur im Bereich von -40 °C bis +100 °C, bevorzugt im Bereich von 0 bis +80 °C, besonders bevorzugt im Bereich von +20 °C bis +80 °C durchgeführt. Eine weitere erfindungsgemäße Ausführungsform ist die Durchführung der Reaktion unter Bestrahlung durch Mikrowellen, welche mit dem Vorteil eines schnelleren Umsatzes einhergeht.

In einer bevorzugten Ausführungsform wird zunächst eine Portion der Organomagnesiumverbindung zur Mischung aus Eisenverbindung, Lösungsmittel und stickstoffhaltigem Additiv oder Cosolvens zugegeben und der restliche Anteil Organomagnesiumverbindung langsam zugetropft. Bei einer weiteren Ausführungsform erfolgt die Zugabe der Organomagnesiumverbindung auf einmal. Letzteres kann insbesondere bei fluorsubstituierten organischen Resten der Organomagnesiumverbindung vorteilhaft sein.

Durch das erfindungsgemäße Verfahren konnten somit erstmalig eine Vielzahl von verschiedenartig substituierten Chlorstyrolen mit Organomagnesium-Verbindungen gekuppelt werden.

## <u>Beispiele</u>

**<u>Beispiel 1</u>** Kupplung von 2-Chlorstyrol mit 4-Dimethylaminophenylmagnesiumbromid

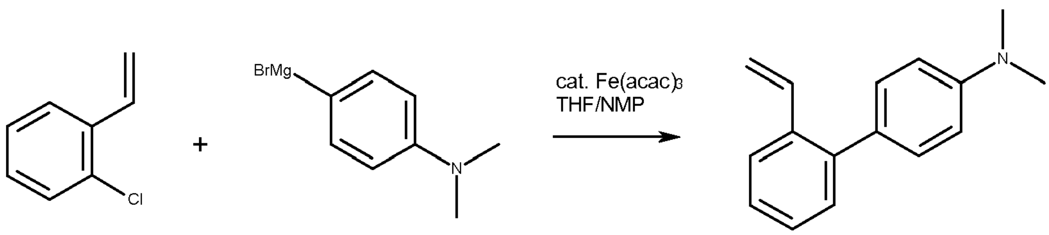


Unter Schutzgas wurden 17.7 mg (5 mol%, 0.05 mmol) Eisen(II)-acetylacetonat in 4.4 ml eines Gemisches von Tetrahydrofuran und NMP (v/v 10:1) aufgenommen. Bei 30° C wurden 138.6 mg (1.0 mmol) 2-Chlorstyrol zugegeben.

Dann wurden 3 ml einer 0.5 M Lösung von 4-Dimethylaminophenylmagnesiumbromid in THF (ca. 1.5 mmol) zugegeben. Nach zweistündigem Nachrühren wurde das Reaktionsgemisch mit 5 ml einer gesättigten Natriumcarbonat-Lösung verwässert und dreimal mit je 5 ml Ethylacetat extrahiert. Die vereinigten organischen Phasen wurden über Magnesiumsulfat getrocknet, am Rotationsverdampfer eingeengt und das erhaltene Rohprodukt durch Gaschromatographie analysiert. Die Ausbeute betrug 146 mg (0.65 mmol, 65% d. Th.) 2'-Vinyl-4-dimethylaminobiphenyl.

**Beispiele 2 bis 15**

Weitere Kupplungen von Chlorstyrolen mit Arylgrignard-Verbindungen wurden durchgeführt, wobei die Durchführung analog zu Beispiel 1, jedoch mit den in Tabelle 1 aufgeführten Reaktanden erfolgte. Die Einzelausbeuten sind nicht optimiert.

Tabelle 1

| Beispiel Nr. | Chlorstyrol | Grignard-Verbindung | Produkt | Ausbeute (% d. Th.) |
|---|---|---|---|---|
| 2 | 1-Chlor-4-isopropenylbenzol | 4-Fluorphenyl-magnesiumbromid | 4-Fluor-4'-isopropenyl biphenyl | 93 |
| 3 | 2-Chlorstyrol | 4-Methoxyphenyl-magnesiumbromid | 4-Methoxy-2'-vinyl-biphenyl | 71 |
| 4 | 2-Chlorstyrol | 4-Fluorphenyl-magnesiumbromid | 4-Fluor-2'-vinylbiphenyl | 83 |
| 5 | 4-Dimethylamino-2-chlor-styrol | 4-Fluorphenyl-magnesiumbromid | 3-Dimethylamino-4'-fluor-2-vinylbiphenyl | 72 |
| 6 | 2-Chlorstyrol | 4-Fluor-3-methylphenyl-magnesiumbromid | 4-Fluor-3-methyl-4'-vinylbiphenyl | 83 |
| 7 | 2-Chlor-3-methoxystyrol | 4-Methoxyphenyl-magnesiumbromid | 2,4'-Dimethoxy-6-vinylbiphenyl | 91 |
| 8 | 2-Chlor-3-methoxystyrol | 4-Fluorphenyl-magnesiumbromid | 2-Methoxy-4'-fluor-2-vinylbiphenyl | 70 |
| 9 | 2-Chlor-3-methoxystyrol | 4-Dimethylaminophenyl-magnesiumbromid | 2'-Methoxy-4-dimethyl-amino-6'-vinylbiphenyl | 73 |
| 10 | 2-Chlorstyrol | 4-Trifluormethoxyphenyl-magnesiumbromid | 4-Trifluormethoxy-2'-vinylbiphenyl | 83 |
| 11 | 2-Chlorstyrol | 1,3-Benzo-dioxol-5-yl-magnesiumbromid | 5-(2-Vinylphenyl)-benzo[d][1,3]dioxol | 82 |
| 12 | 2-Chlor-4,5-dimethoxystyrol | 1,3-Benzo-dioxol-5-yl-magnesiumbromid | 5-(4,5-Dimethoxy-2-vinyl-phenyl)benzo[d][1,3]dioxol | 69 |
| 13 | 1-Chlor-3-isopropenylbenzol | 4-Fluorphenyl-magnesiumbromid | 4-Fluor-3'-isopropenyl biphenyl | 85 |
| 14 | 2,4-Dichlorstyrol | Phenylmagnesiumbromid | 5-Chlor-2-vinylbiphenyl | 69 |
| 15 | 2-Chlorstyrol | Phenylmagnesiumbromid | 2-Vinylbiphenyl | 89 |

**Vergleichsbeispiele 1 bis 3**

Die Kupplung von elektronenreichen Arylchloriden mit Arylgrignard-Verbindungen wurde versucht, wobei die Durchführung analog zu Beispiel 1 jedoch mit den in Tabelle 2 aufgeführten Reaktanden erfolgte. Es zeigte sich, dass in Abwesenheit der Alkenylgruppe keine Kupplung erfolgt.

Tabelle 2

| Vergleichs-beispiel Nr. | | Grignard-Verbindung | Produkt | Ausbeute (% d. Th.) |
|---|---|---|---|---|
| 1 | Chlorbenzol | Phenylmagnesiumchlorid | Biphenyl | 1 |
| 2 | 2-Chloranisol | Phenylmagnesiumchlorid | 2-Methoxybiphenyl | 0 |
| 3 | 2-Chlortoluol | Phenylmagnesiumchlorid | 2-Phenyltoluol | 0 |

**Beispiele 16 bis 26**

Kupplungen von Chlorstyrolen mit Alkylgrignard-Verbindungen wurden durchgeführt, wobei die Durchführung analog zu Beispiel 1 jedoch mit den in Tabelle 3 aufgeführten Reaktanden erfolgte. Die Einzelausbeuten sind nicht optimiert.

Tabelle 3

| Beispiel Nr. | Chlorstyrol | Grignard-Verbindung | Produkt | Ausbeute (% d. Th.) |
|---|---|---|---|---|
| 16 | 2-Chlorstyrol | 1-Decylmagnesiumbromid | 2-Decylstyrol | 94 |
| 17 | 2-Chlorstyrol | 1-Undecylmagnesium-bromid | 2-Undecylstyrol | 85 |
| 18 | 4-Isopropenyl-chlorbenzol | 1-Decylmagnesiumbromid | 1-Isopropenyl-4-decylstyrol | 71 |
| 19 | 4-Isopropenyl-chlorbenzol | 1-Undecylmagnesium-bromid | 1-Isopropenyl-4-undecylstyrol | 70 |
| 20 | 2-Chlorstyrol | 2-Ethylhexyl-1-magnesiumbromid | 2-(2-Ethylhexyl)-styrol | 80 |
| 21 | 2-Chlorstyrol | 2,2-(Propylendioxy)-ethyl-1-magnesiumbromid | 2-(2,2-Propylendioxy-)ethylstyrol | 97 |
| 22 | 2-Chlorstyrol | Cyclopropylmagnesium-bromid | 2-Cyclopropylstyrol | 43 |
| 23 | 4-Chlorstilben | Nonylmagnesiumbromid | 4-Nonylstilben | 60 |
| 24 | 4-(Propen-1-yl)-1-chlorbenzol | Nonylmagnesiumbromid | 4-Nonyl-1-(propen-1-yl)-benzol | 63 |
| 25 | 2-Chlorstyrol | 2-Cyclohexylethyl-magnesiumbromid | 2-(2-Cyclohexyl)-ethylstyrol | 75 |
| 26 | 2-Chlorstyrol | Dec-9-en-1-ylmagnesium-bromid | 2-(Dec-9-en-1-yl)-styrol | 71 |
| 27 | 2-Chlorstyrol | [6-(Tetrahydro-2H-pyran-2-yl)oxy]hexylmagnesium-bromid | 2-[6-(Tetrahydro-2H-pyran-2-yl)oxy]hexyl-styrol | 77 |
| 28 | 2-Chlor-3-methoxystyrol | Dec-9-en-1-ylmagnesium-bromid | 2-(Dec-9-en-1-yl)-3-methoxystyrol | 70 |
| 29 | 2-Chlor-3-methoxystyrol | 2,2-(Propylendioxy)-ethyl-1-magnesiumbromid | 3-Methoxy-2-(2,2-propylendioxy)ethyl-styrol | 70 |
| 30 | 2-Chlor-4,5-(me-thylendioxy)-styrol | Decylmagnesiumbromid | 2-Decyl-4,5-(methy-lendioxy)-styrol | 73 |

(fortgesetzt)

| Beispiel Nr. | Chlorstyrol | Grignard-Verbindung | Produkt | Ausbeute (% d. Th.) |
|---|---|---|---|---|
| 31 | 2-Chlor-4,5-(methylendioxy)-styrol | 2,2-(Propylendioxy)-ethyl-1-magnesiumbromid | 2-(2,2-Propylendioxy)-ethyl-4,5-(methylendioxy)-styrol | 82 |
| 32 | 4,5-Dimethoxy-2-chlorstyrol | 2,2-(Propylendioxy)-ethyl-1-magnesiumbromid | 2-(2,2-Propylendioxy)-ethyl-4,5-dimethoxystyrol | 73 |
| 33 | 1-Chloro-4-[3-(4-chlorphenyl)-1-buten-1-yl]-benzol | Decylmagnesiumbromid | 4-[3-(4-Chlorphenyl)-1-buten-1-yl]-1-decylbenzol | 61 |

**Beispiel 34**

Die Kupplung von 2-Chlorstyrol mit Decylmagnesiumbromid wurde wie in Beispiel 16 beschrieben durchgeführt, allerdings bei einer Reaktionstemperatur von 55° C. Die isolierte Ausbeute an 2-Decylstyrol konnte dabei auf 97% gesteigert werden.

**Beispiel 35**

Die Kupplung von 2-Chlorstyrol und 4-Fluorphenylmagnesiumbromid wurde wie in Beispiel 4 beschrieben durchgeführt, allerdings wurde die Reaktionsmischung mit Mikrowellen aus einem handelsüblichen Mikrowellengerät beaufschlagt. Dabei stieg die Reaktionstemperatur auf 80° C an. Nach 15 min Reaktionszeit betrug die Ausbeute 85%.

**Patentansprüche**

**1.** Verfahren zur Herstellung organischer Verbindungen der allgemeinen Formel (I)

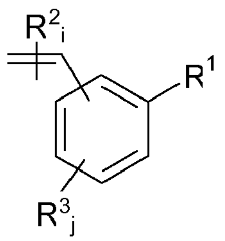

(I),

worin

i für 0, 1, 2 oder 3, bevorzugt für 0 oder 1 steht,
j für 0, 1, 2, 3 oder 4, bevorzugt für 0 oder 1 steht,
$R^2$ unabhängig voneinander ausgewählt ist aus unsubstituiertem, oder mit Fluor, $NR^4_2$, $C_1$-$C_6$-Alkyl oder $C_1$-$C_6$-Alkoxy-substituiertem $C_1$-$C_6$-Alkyl, $C_6$-$C_{10}$-Aryl oder $C_7$-$C_{11}$-Aralkyl, wobei jedes $R^4$ unabhängig voneinander, für $C_1$-$C_6$-Alkyl, $C_7$-$C_{10}$-Aryl oder $C_7$-$C_{11}$-Aralkyl stehen kann,
$R^3$ unabhängig voneinander ausgewählt ist aus Fluor, Chlor, $NR^4_2$, unsubstituiertem, oder mit $NR^4_2$ oder $C_1$-$C_6$-Alkoxy-substituiertem $C_1$-$C_6$-Alkyl, sowie unsubstituiertem, oder mit Fluor, $NR^4_2$ oder $C_1$-$C_6$-Alkoxy-substituiertem $C_1$-$C_6$-Alkoxy oder $C_7$-$C_{11}$-Aralkyl, wobei $R^4$ jeweils unabhängig voneinander die oben angegebenen Bedeutungen hat, wobei zwei oder mehr der Substituenten $R^2$ und/oder $R^3$ zusammen ein aromatisches, heteroaromatisches, aliphatisches oder heteroaliphatisches Ringsystem bilden, unter der Bedingung, dass die $R^2$ tragende Vinylfunktion nicht Bestandteil eines solchen Ringsystems ist, sofern dieses aromatisch oder heteroaromatisch ist, und
$R^1$ ein unsubstituierter, oder mit Fluor, $C_1$-$C_{10}$-Alkyl, $C_1$-$C_6$-Alkoxy, oder $NR^4_2$, substituierter $C_1$-$C_{15}$-Alkyl, $C_3$-$C_7$-Cycloalkyl, $C_2$-$C_{15}$-Alkenyl, $C_3$-$C_7$-Cycloalkenyl, $C_2$-$C_{15}$-Alkinyl, $C_1$-$C_6$-Alkoxy-, $C_7$-$C_{15}$-Aralkyl-, oder

$C_6$-$C_{10}$-Aryl-Rest, oder ein entsprechend substituierter oder unsubstituierter aliphatischer oder aromatischer $C_3$-$C_{12}$-Heterocyclus ist, wobei $R^4$ jeweils unabhängig voneinander die oben angegebenen Bedeutungen hat,

durch Umsetzung von Chlorstyrolen der allgemeinen Formel (II)

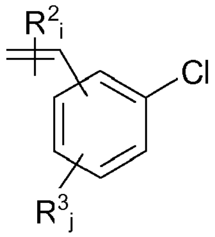

(II),

worin
$R^2$, $R^3$, i und j die für Formel (I) beschriebenen Bedeutungen haben,
mit Organomagnesiumverbindungen der formalen Zusammensetzung (III)

$$[M^+]_n\ [R_mMgX_kY_1] \qquad \text{(III)},$$

worin

$R^1$ die für Formel (I) beschriebene Bedeutung hat,
M für Lithium, Natrium oder Kalium steht,
X für Fluor, Chlor, Brom oder Iod, bevorzugt Chlor oder Br Chlor, Brom, ganz besonders bevorzugt für Brom steht,
Y für Fluor, Chlor, Brom oder Iod, bevorzugt Chlor oder Br Chlor, Brom, ganz besonders bevorzugt für Brom steht,
n für 0, 1, 2, 3 oder 4, bevorzugt 0 oder 1, ganz besonders bevorzugt für 0 steht,
m für 1, 2, 3, 4, 5 oder 6, bevorzugt 1 oder 2, ganz besonders bevorzugt für 1 steht,
k für 0, 1, 2, 3 oder 4, bevorzugt 0, 1 oder 2, ganz besonders bevorzugt für 0 oder 1 steht,
l ür 0, 1, 2, 3 oder 4 bevorzugt 0, 1 oder 2, ganz besonders bevorzugt für 0 oder 1 steht,

und gleichzeitig die Beziehung

$$n + 2 = m + k + 1$$

gilt,
**dadurch gekennzeichnet, dass** die Umsetzung in Gegenwart von Eisenverbindungen, bevorzugt Eisen(II)- oder Eisen(III)-Verbindungen durchgeführt wird.

**2.** Verfahren nach Anspruch 1, **dadurch gekennzeichnet, dass** $R^2$ unabhängig voneinander ausgewählt ist aus $C_1$-$C_6$-Alkyl, besonders bevorzugt Methyl, Ethyl oder Propyl, $C_6$-$C_{10}$-Aryl, besonders bevorzugt Phenyl oder Naphthyl und $C_7$-$C_{11}$-Aralkyl.

**3.** Verfahren nach einem der vorhergehenden Ansprüche, **dadurch gekennzeichnet, dass** $R^3$ unabhängig voneinander ausgewählt ist aus Chlor, $NR^4_2$, wobei jedes $R^4$ unabhängig voneinander, die Bedeutung gemäß Anspruch 1 hat, $C_1$-$C_6$-Alkyl, besonders bevorzugt Methyl, Ethyl oder Propyl, $C_1$-$C_6$-Alkoxy, $C_6$-$C_{10}$-Aryl, besonders bevorzugt Phenyl oder Naphthyl und $C_7$-$C_{11}$-Aralkyl.

**4.** Verfahren nach einem der vorhergehenden Ansprüche, **dadurch gekennzeichnet, dass** $R^1$ ein unsubstituierter, oder mit Fluor, $C_1$-$C_4$-Alkyl, $C_1$-$C_4$-Alkoxy oder $NR^4_2$, substituierter $C_1$-$C_{11}$-Alkyl, $C_3$-$C_7$-Cycloalkyl, $C_2$-$C_{11}$-Alkenyl, $C_3$-$C_7$-Cycloalkenyl, $C_2$-$C_{11}$-Alkinyl, $C_7$-$C_{12}$-Aralkyl-, oder $C_6$-$C_{10}$-Aryl-Rest, oder ein entsprechend substituierter oder unsubstituierter aliphatischer oder aromatischer $C_3$-$C_8$-Heterocyclus, wobei $R^4$ jeweils unabhängig voneinander $C_1$-$C_6$-Alkyl ist.

**5.** Verfahren nach einem der vorhergehenden Ansprüche, **dadurch gekennzeichnet, dass** als Eisenverbindung Eisen(II)chlorid, Eisen(III)chlorid, Eisen(II)acetylacetonat, Eisen(III)-acetylacetonat, Eisen(II)acetat, Eisen(III)acetat, Eisen(II)bromid, Eisen(III)bromid, Eisen(II)fluorid, Eisen(III)fluorid, Eisen(II)iodid, Eisen(III)iodid, Eisen(II)sulfat, Eisen (II)-trifluoracetat, Eisen(II)trifluormethansulfonat, Eisen(III)trifluormethansulfonat, Eisen(III)-chlorid-TMEDA-Komplex oder eine Mischung dieser Verbindungen eingesetzt wird.

**6.** Verfahren nach einem der vorhergehenden Ansprüche, **dadurch gekennzeichnet, dass** die Eisenverbindung in substöchiometrischer Menge, vorzugsweise in einer Menge von 0,01 bis 50 mol%, besonders bevorzugt von 0,01 bis 10 mol% bezogen auf die Verbindung der allgemeinen Formel (II), eingesetzt wird.

**7.** Verfahren nach einem der vorhergehenden Ansprüche, **dadurch gekennzeichnet, dass** als Organomagnesiumverbindung der formalen Zusammensetzung (III) Organomagnesiumchloride $R^1MgCl$ oder Organomagnesiumbromide $R^1MgBr$ eingesetzt werden, wobei $R^1$ die in Anspruch 1 angegebene Bedeutung hat.

**8.** Verfahren nach einem der vorhergehenden Ansprüche, **dadurch gekennzeichnet, dass** die Reaktion in einem Lösungsmittel durchgeführt wird, welches ein oder mehrere stickstoffhaltige Additive, ausgewählt aus Trialkylaminen, vorzugsweise Triethylamin, Ethyldiisopropylamin oder N,N,N',N'-Tetramethylethylendiamin, N-haltigen aliphatischen Heterocyclen, vorzugsweise 1,4-Diazabicyclo[2.2.2]octan oder Spartein, Alkylamide, cyclischen Alkylamiden (Lactamen), vorzugsweise N-Methyl-2-pyrrolidon, Cycloalkylaminen, vorzugsweise 1,2-Diaminocyclohexan, Cycloalkyldiaminen, Alkyliminen, Cycloalkyliminen, Anilin-Derivaten, vorzugsweise N,N-Dimethylanilin, Harnstoffen, Urethanen oder stickstoffhaltigen Heteroaromaten, vorzugsweise Pyridin oder Phenanthrolin enthält.

**9.** Verfahren nach einem der vorhergehenden Ansprüche, **dadurch gekennzeichnet, dass** das stickstoffhaltige Additiv in einer Menge von 0,1 bis 50 Vol.-%, bevorzugt von 1 bis 20 Vol.-% und ganz besonders bevorzugt von 5-15 Vol.-% bezogen auf das bei der Umsetzung verwendete Lösungsmittel, eingesetzt wird.

**10.** Verfahren nach einem der vorhergehenden Ansprüche, **dadurch gekennzeichnet, dass** das Verfahren in einem aprotischen organischen Lösungsmittel, bevorzugt in Tetrahydrofuran, 2-Methyltetrahydrofuran, 1,4-Dioxan, Methyltert-butylether, Diethylether, 1,2-Dimethoxyethan, Diisopropylether, Dipropylether, Dibutylether, Cyclopentylmethylether, Diethylenglycoldimethylether, Triethylenglycoldimethylether, Tetraethylenglycoldimethylether, Diethylenglycoldibutylether, Dimethylcarbonat, 1,1,2,2-Tetraethoxyethan, 1,1,2,2-Tetramethoxyethan oder einer Mischung derselben, durchgeführt wird.

**11.** Verfahren nach einem der vorhergehenden Ansprüche, **dadurch gekennzeichnet, dass** die Reaktion unter Bestrahlung mit Mikrowellen durchgeführt wird.

Europäisches Patentamt
European Patent Office
Office européen des brevets

# EUROPÄISCHER RECHERCHENBERICHT

Nummer der Anmeldung
EP 12 17 0697

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | SAMET GÜLAK ET AL: "Chlorostyrenes in Iron-Catalyzed Biaryl Coupling Reactions", ANGEWANDTE CHEMIE INTERNATIONAL EDITION, Bd. 51, Nr. 6, 6. Februar 2012 (2012-02-06), Seiten 1357-1361, XP55038884, ISSN: 1433-7851, DOI: 10.1002/anie.201106110<br>* das ganze Dokument *<br>----- | 1-11 | INV.<br>C07C209/68<br>C07B37/04<br>C07B49/00<br>C07D317/50<br>C07C1/32<br>C07C41/30<br>C07C17/263 |
| | | | RECHERCHIERTE SACHGEBIETE (IPC) |
| | | | C07C<br>C07B<br>C07D |

Der vorliegende Recherchenbericht wurde für alle Patentansprüche erstellt

| Recherchenort | Abschlußdatum der Recherche | Prüfer |
|---|---|---|
| Den Haag | 21. September 2012 | Zervas, Brigitte |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................
& : Mitglied der gleichen Patentfamilie, übereinstimmendes Dokument

2

EPO FORM 1503 03.82 (P04C03)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006125511 A, Lietzau


### In der Beschreibung aufgeführte Nicht-Patentliteratur


- **NIWA et al.** *Org. Lett.,* 2008, vol. 10, 4639
- **TORBORG et al.** *Chem. Eur. J.,* 2009, vol. 15, 1329
- **KOCHI et al.** *J. Am. Chem. Soc.,* 1971, 1487
- **FÜRSTNER et al.** *Angew. Chem. Int. Ed.,* 2002, vol. 41, 609
- **NAKAMURA et al.** *J. Am. Chem. Soc.,* 2004, 3686
- **KNOCHEL et al.** *Synlett,* 2001, 1901
- **CAHIEZ et al.** *Angew. Chem. Int. Ed.,* 2007, 4364